# EUROPEAN PATENT APPLICATION

(11) **EP 4 699 711 A1**
(43) Date of publication of application: **25.02.2026**
(21) Application number: 25197254.3
(22) Date of filing: 21.08.2025
(51) Int. Cl.: B06B 1/06

(54) **BACKING WITH GRAPHENE FOR AN ULTRASOUND TRANSDUCER**

(30) Priority: 23.08.2024 US 202418813098
(71) Applicant: Siemens Medical Solutions USA, Inc., Malvern, PA 19355-1406 (US)
(72) Inventor: ABOTHU, Isaac, Sammamish, WA, 98074 (US); CHA, Phong, Carnation, WA, 98014 (US); CHO, Jeongrae, Issaquah, WA, 98029 (US); LEE, SeungHee, Buk-gu, Pohang-si, Gyeongsangbuk-do (KR); LU, Xuan-Ming, Bellevue, WA, 98008 (US); DRESCHEL, William R., State College, PA, 16803 (US)
(74) Representative: HKW Intellectual Property PartG mbB

(57) **Abstract**

For ultrasound transducers. graphene (174), such as flakes or sheets (174B), is used in backers (170) with a matrix of polymer (172). The graphene (174) provides good attenuation at low frequencies and thermal conductivity.

## Description

The present embodiments relate to ultrasound transducers. Ultrasound transducers include a piezoelectric (PZT) layer stacked on a backing block. Ultrasound backing blocks are typically made with a composite of a polymer (epoxy or silicone) matrix supporting metallic particles. These types of backers are often not thermally conductive and have low attenuation at low frequencies or poor mechanical strength.

High thermal conductivity may be provided by a porous graphite or metal foam with voids filled with polymer. These backers where the thermally conductive material forms the matrix or supporting structure have low attenuations at low frequencies. Some unfilled voids may potentially induce imaging artifacts.

### SUMMARY

By way of introduction, the preferred embodiments described below include methods, systems, backings, and components for ultrasound transducers. Graphene, such as flakes or sheets, is used in backers with a matrix of polymer. The graphene provides good attenuation at low frequencies and thermal conductivity.

In a first aspect, a transducer array system includes an array of ultrasound transducer elements, and a backing having a polymer framework with one or more sheets of graphene supported by the polymer framework.

In a second aspect, a method is provided for forming an ultrasound backing block. Graphene sheets are combined with a polymer. The polymer is cured. The polymer as cured is a support matrix supporting the graphene flakes or sheets.

In a third aspect, an ultrasound transducer backing block includes a matrix of cured polymer, the cured polymer comprising soft polymer, and flakes of graphene in the matrix. The flakes of graphene held in place in the matrix by the cured polymer around the graphene.

Further aspects and features are summarized below in the Illustrative Embodiments. Different aspects and/or features may be used in various combinations. Aspects and/or features in one context (e.g., system, backing, or method) may be used in another context.

The present invention is defined by the following claims, and nothing in this section should be taken as a limitation on these claims. Further aspects and advantages of the invention are discussed below in conjunction with the preferred embodiments and may be later claimed independently or in combination. Different embodiments may achieve or fail to achieve different objects or advantages.

### BRIEF DESCRIPTION OF THE DRAWINGS

The components and the figures are not necessarily to scale, emphasis instead being placed upon illustrating the principles of the invention. Moreover, in the figures, like reference numerals designate corresponding parts throughout the different views.
Figure 1 is a cross-section view of an example transducer stack using a backing with integrated graphene flakes;
Figures 2A and 2B are bar charts showing comparative attenuation and thermal conductivity, respectively, for various backings including a backing with integrated graphene;
Figures 3-7 are cross-sectional views of example backings with sheets of graphene;
Figure 8 is a perspective view of an example backing formed as a roll of graphene supported by a polymer matrix; and
Figure 9 is a flow chart diagram of one embodiment of a method for forming a backing with integrated graphene.

### DETAILED DESCRIPTION OF THE DRAWINGS AND PRESENTLY PREFERRED EMBODIMENTS

A backing block structure includes graphene. Graphene has high mechanical strength and high thermal conductivity, such as about 1000W/mK along a sheet. Graphene also has low velocity and high attenuation in the Z or thickness dimension due to the weak (Van der Waals) force between graphene layers. The unique properties of graphene may be utilized to achieve a mechanically stable backing with high attenuation and high thermal conductivity.

Instead of using a block of graphite with voids (e.g., foam matrix), the graphene is integrated in a polymer matrix type of backing block. The resulting backing utilizes the mechanical and thermal properties of graphene to provide good attenuation, thermal conductivity, and mechanical strength in a balanced approach. The graphene may be integrated in the polymer-based backing with metal particles as micro-flakes so that the backing combines high impedance, high attenuation, and high thermal conductivity as compared to many existing backers. The graphene may be integrated in the polymer-based backing as graphene sheets potted with the polymer-based backing to overcome graphene sheets bonding issues.

The composite backing of a polymer matrix with integrated graphene may be used for various transducers, such as one dimensional, 1.5 dimensional, and two-dimensional transducers. Thermal quality is better than other polymer-based backings. Imaging quality may be better than metal foam-based backings. Better low frequency operation may be provided.

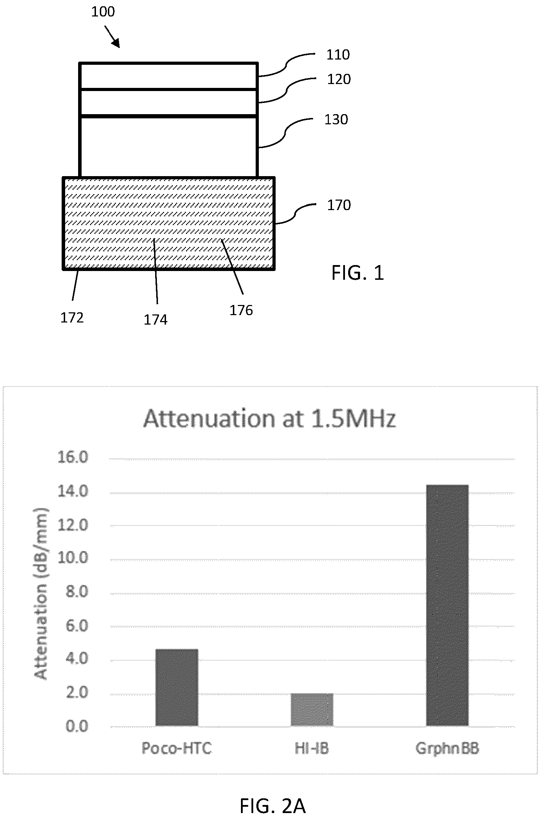

Figure 1 is a cross-section view of one implementation of a transducer array system. The cross-section is in the depth-elevation dimension where depth is vertical on the drawing and elevation is horizontal on the drawing. The elements of the array 100 are distributed along the azimuth dimension (into or out of the drawing page). The transducer array system includes a backing block 170 formed with a polymer matrix 172 supporting flakes of graphene 174 and optional powder or particles 176, such as a metal powder for impedance matching.

The transducer array system is used for an ultrasound transducer probe, such as in a handheld probe for scanning from an exterior of a patient or an intra-cavity (e.g., TEE or TTE) or catheter-based probe for scanning from within a patient. The system includes a one or multi-dimensional transducer array 100. As a multi-dimensional array 100, the array 100 may be a 1.25, 1.5, 1.75, or 2D array with a distribution of elements in both azimuth and elevation.

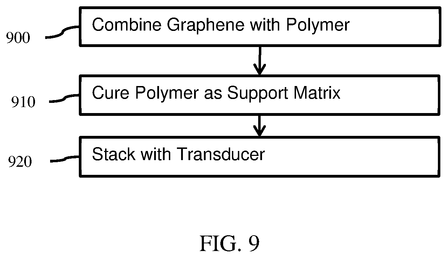

The transducer array system includes matching layers 110, 120, an array layer (e.g., piezoelectric (PZT) layer 130), and an acoustic backer (backing block of acoustic attenuation material) 170. Additional, different, or fewer layers may be included, such as a ground electrode and signal electrodes. In another example, integrated circuits or chips connected to the backing layer are provided. As another example, one of the matching layers 110, 120 is not included, or additional matching layers are included. In one example, a lens and/or housing is provided around the transducer array system or adjacent to the second acoustic matching layer 110. In other examples, a layer of flexible circuit material layer connects between the backing block 170 and the PZT layer 130, to a side of the PZT layer 130, at the back of the backing block 170 opposite the PZT layer 130, and/or another location for routing signals between the PZT layer 130 and the ultrasound scanner. The transducer array system and corresponding probe are formed using the method of Figure 9 or another method.

The matching layers 110 and 120 are ¼ wavelength thickness layers of material. Multiple layers for a gradual change in acoustic impedance may be used, but only one matching layer is provided in other implementations. The second matching layer 110 provides a transition in acoustic impedance between the patient, lens, or other material and the first matching layer 120. Similarly, the first matching layer 120 provides an acoustic impedance transition from the second matching layer 110 to the piezoelectric or other transducer array layer 130.

The array layer is shown as a PZT layer 130 but may include the first matching layer 120. The PZT layer 130 is a slab or plate of PZT material. A solid PZT may be used. Single or poly-crystal PZT material may be used. In other embodiments, a composite of piezoelectric and epoxy or another polymer is used. Microelectromechanical (capacitive membrane) elements may be used instead of PZT. Piezoelectric examples are used herein, so the array layer will be referenced as the PZT layer 130.

Once diced, the PZT layer 130 forms the array 100 of transducer elements. The transducer elements of the array 100 or PZT layer 130 are distributed in a grid over one or two dimensions. The PZT layer 130 may form a 1D array with the transducer elements distributed in a line, whether straight or curved. The PZT layer 130 may form a multi-dimensional array 100 where the elements are distributed in two dimensions (azimuth and elevation).

The transducer array 100 is an array of PZT elements. The elements transduce between acoustic and electrical energies, such as an array of transducer elements formed from PZT material. Kerfs separate the elements of the PZT layer 130. The kerfs may also separate the first acoustic matching layer 120. In other embodiments, the kerfs separate the second matching layer 110. In yet other embodiments, the kerfs do not extend through the first acoustic matching layer 120. The array is flat, concave, or convex.

Each of the transducer elements of the array includes at least two electrodes, a ground electrode and a signal electrode. The signal electrodes are separated by the kerfs with the PZT layer 130, such as being electrodes deposited on the PZT layer 130. The elements transduce between electrical and acoustical energies. The electrical energy generated by the PZT or provided to the PZT is provided on the signal electrode separate from the ground electrode. The signal electrodes 165 connect with the signal conductors of the backing block, signal conductors on a flexible circuit, or another conductor forming a signal path to and from the array 100.

The array 100 and corresponding PZT layer 130 are positioned adjacent to the backing block 170. One side of the backing block 170 contacts (e.g., asperity contact or through solder) with a side of the array 100 or flexible circuit material between the PZT layer 130 and the backing block 170. Other layers may separate the PZT layer 130 from the backing block 170, such as bonding material.

The backing block 170 is a backing for the ultrasound transducer formed by the array 100. The backing block 170 may be a single molded or formed (e.g., machined) block for all the elements. Alternatively, separate backing blocks 170 are provided for different groups of elements and/or at different parts of the block.

The backing block 170 is shaped and sized to mate with the array 100. The dimensions of the backing block 170 along azimuth and lateral dimensions of the array 100 may match the array 100 or extend beyond the array 100. Figure 1 shows the elevation extent of the backing block 170 extending beyond the array 100. The depth of the backing block 170 may be greater than the ¼ of the longest wavelength for which the array 100 is to be used. In one embodiment, the depth is greater than the depth of the PZT layer 130. The backing block 170 may include protrusions or indentations for mating with the PZT layer 130. Epoxy or other bonding agent or glue may be used to connect the backing block 170 with the PZT layer 130 in asperity contact or with intervening layers. Clips, fasteners, or other structures may be added to mate or connect the backing block 170 to the array 100. Once aligned, the backing block 170 (e.g., molded block of acoustic absorber) is bonded or fixed to the PZT layer 130.

The backing block 170 is an acoustic absorber. The backing block 170 is formed from a polymer, such as epoxy or silicone, another thermosetting or thermoplastic polymer, or another acoustic absorber. This polymer is combined with other materials, such as forming a composite of different materials. The different materials may be mixed, such as a composite backing.

A matrix or base material is the polymer 172, but other bonding agents may be used, such as elastomers, for the acoustic attenuating material. The backing block 170 is formed from a support structure of cured polymer 172, such as epoxy with the thermoset and thermoplastic components mixed to chemically cure. Heat, pressure, or other environmental control may be used to form the cured epoxy or another cured polymer. As a support structure, the cured polymer 172 surrounds, holds, and/or supports any filler materials, such as graphene 174 and optional powder or particles 176. A matrix of cured polymer 172, such as cured epoxy, forms the majority by volume and/or weight of the backing block 170. The backing block 170 is a polymer framework with graphene 174 supported by the polymer framework. A polymer-based composite has the graphene 174 potted inside the polymer framework. A continuous matrix of polymer 172 is the support structure or framework. The integrated graphene 174 is within the continuous matrix.

Any fillers, such as for altering the acoustic attenuation, may be mixed with the polymer before curing, added to a framework where the liquid polymer is poured around the filler and cured, and/or stacked with cured pieces of polymer.

In one implementation, a metal powder 176 is included as a filler. For example, Aluminic (AIN) particles and/or tungsten powder 176 are included or supported by the polymer matrix. The metal powder 176 may be used for tuning impedance but also may have attenuation and/or thermal effects. Other powders or particles 176, such as rubber (e.g., RBT or silicone), may be included.

The backing block 170 includes graphene 174. Graphene 174 has high mechanical strength and high thermal conductivity (~1000W/mK) across a sheet or flake, nevertheless having a low velocity and high attenuation in thickness due to the weak (Van der Waals) force between graphene layers. The unique properties of graphene may be utilized to achieve high attenuation and higher thermal conductivity in a mechanically stable backing block 170.

A filler of graphene 174 is added to the polymer matrix. The polymer 172 supports the graphene 174. The graphene 174 is in the matrix, so the graphene 174 is held in place in the matrix by the cured polymer 172 around the graphene 174.

In the implementation shown in Figure 1, the graphene 174 has a form of flakes, as flowers or chips. The flakes are of uniform or varying sizes. For example, most of the flakes are 5-500 um as the maximum dimension. Each flake is formed from two or more layers of graphene. The large particle size (e.g., about 500 um) of the graphene flakes and weak bonding between graphene sheets forming the flakes contribute to high attenuation at low frequencies (e.g., 1-2 MHz). In one implementation, most of the flakes are 400-500 um as the maximum dimension. Flakes of graphene 174 with multilayers of graphene sheets bonded by the weak Van der Waals force contribute to acoustic energy absorption.

The flakes of graphene 174 are supported within or integrated in the framework or matrix of the polymer 172. For example, micro-flakes of graphene 174 are mixed with the polymer 172 in liquid form (before curing). The polymer 174 may be a hard or soft polymer (i.e., on the shore A range where the cured polymer 172 may be stiff or flexible). In one implementation, the epoxy is soft, such as a shore D of 25 or less (e.g., shore A of 75 or less such as 69). Mid-shore D (e.g., 26-46), or hard-shore D (e.g., 47 or higher) may be used. The soft epoxy (e.g., shore A of 69 or shore D of 22) supporting graphene may provide a substantially better acoustic attenuation than a medium or hard epoxy supporting graphene, such as 3.2 dB/mm at 2.5 MHz for a backing made with DER332 epoxy with a shore D of 78 and graphene flakes, 7.4 dB/mm at 2.5 MHz for a backing made with Hysol epoxy with a shore D of 70 and graphene flakes, and 18.85 dB/mm or more at 2.5 MHz for a backing made with Epo-tek 310M epoxy with a shore D of 22 and graphene flakes. At 1.5 MHz, the soft epoxy with graphene may be 10 dB/mm better than (more than) using medium or hard shore D epoxy. Once cured, the composite of the backing block 170 has the matrix of polymer 172 supporting the flakes of graphene 174 with a uniform or varying distribution within the matrix.

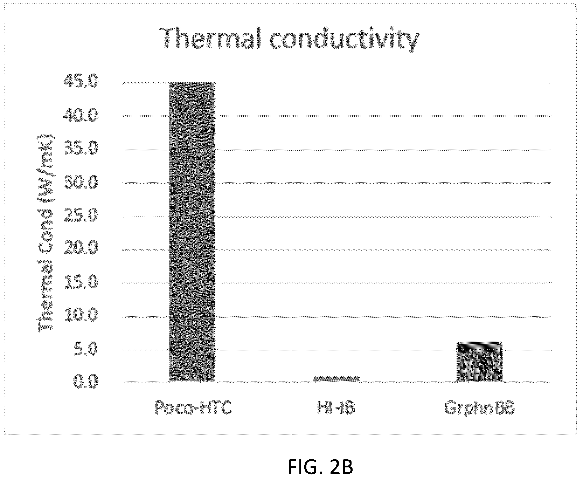

Various ratios of the polymer 172 and graphene 174, with or without other powders or particles 176, may be used. Compositions can be selected for optimal attenuation and thermal conductivity. Figures 2A and 2B show attenuation at 1.5 MHz and thermal conductivity, respectively, for different backing blocks. The graphene backing block 170 (GrphnBB) is formed where the ratios of polymer 172, graphene 174, and metal powder 176 are epoxy 26%, tungsten powder 60%, and graphene flakes or powder 13% by weight. GrphnBB is compared to Poco-HTC (graphite matrix or foam with epoxy void filler) and HI-IB (8% epoxy, 24% silicone rubber powder, 25% aluminum nitride, and 42% tungsten powder by weight). As shown in Figure 2A, the graphene-based composite has a better acoustic attenuation at 1.5 MHz (similar acoustic attenuation to other backers with the best acoustic impedance). As shown in Figure 2B, the graphene-based composite has the second-best thermal conductivity. The graphite matrix backing (Poco-HTC) has the highest thermal conductivity but suffers from low attenuation and high cost. The composite of HI-IB has very low thermal conductivity. The graphene filled epoxy composite-based backing block 170 uses high absorption between graphene sheets to improve the low frequency attenuation (e.g., 10-30 dB/mm more attenuation @1.5-2MHz). The graphene filled epoxy composite provides a good combination of all the desired properties (high acoustic impedance, high attenuation, and high thermal conductivity).

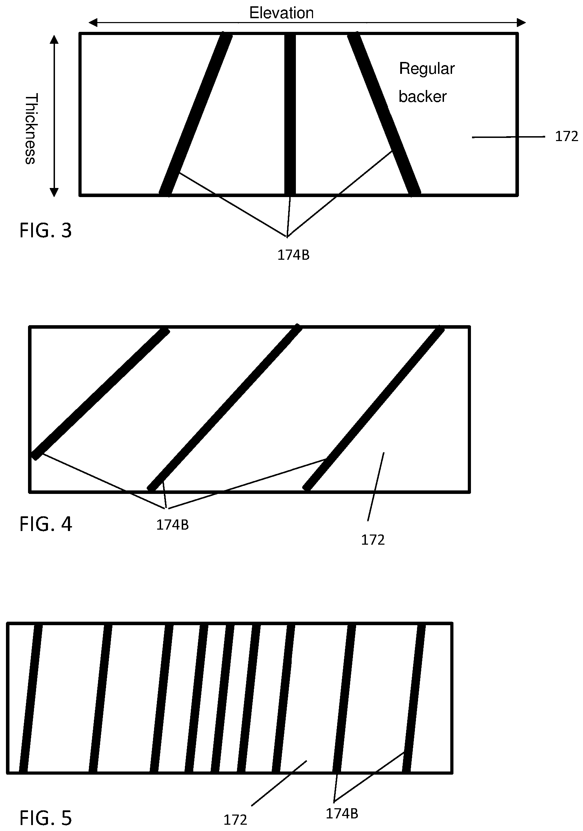

Figures 3-8 show implementations of the polymer backer with graphene filler as sheets 174B of graphene 174. One or more sheets 174B of graphene 174 are within the polymer framework or matrix. The polymer 172 supports or holds the sheets 174B of graphene 174. Graphene sheets 174B (e.g., 0.1mm -1mm) are embedded in the polymer 172, forming a polymer-based backer. The sheets 174B of graphene 174 boost attenuation and thermal conductivity. By using the sheets 174B as filler in the polymer-based backing 170, the weak bonding problem of graphene is less of an issue. The matrix of polymer 172 holds the sheets 174B of graphene 174 in place. Other filler may be included, such as flakes of graphene 174 (see Figure 1) and/or other particles or powders 176.

Directly using the graphene sheets 174B as a backer is not feasible due to the weak bonding of graphene sheets 174B and extreme low velocity that results in strong reflection at the backer interface. Instead, the sheets 174B of graphene 174 are integrated in (e.g., potted) the polymer 172 or another backer matrix. The graphene 174 boosts the thermal conductivity and attenuation, while the polymer 172 of the matrix or support structure limits reflections while holding the sheets 174B in place.

The sheets 174B of graphene 174 are made with layers of single molecular graphene layers bonded by Van der Waals force (weak). In one implementation, each sheet is 0.1-1 mm thick with an area extending substantially side-to-side in the backer 170. "Substantially" in this context is +/- 10% to account for tolerance and/or bridges or gaps for continuation of the polymer matrix. Other thicknesses and/or less area (e.g., extending only part way through the backer 170) may be used. For example, the sheet 174B extends at least halfway between opposing sides. One-quarter or larger area of a cross-section between opposing sides may be used.

The sheets 174B of graphene 174 have high thermal conductivity (e.g., about 1000 W/mK) and good tensile strength along the sheet. In thickness, the velocity and impedance are low (e.g., close to air), and the attenuation is high. These unique properties are utilized to provide good backer performance (high impedance, high attenuation, and high thermal conductivity).

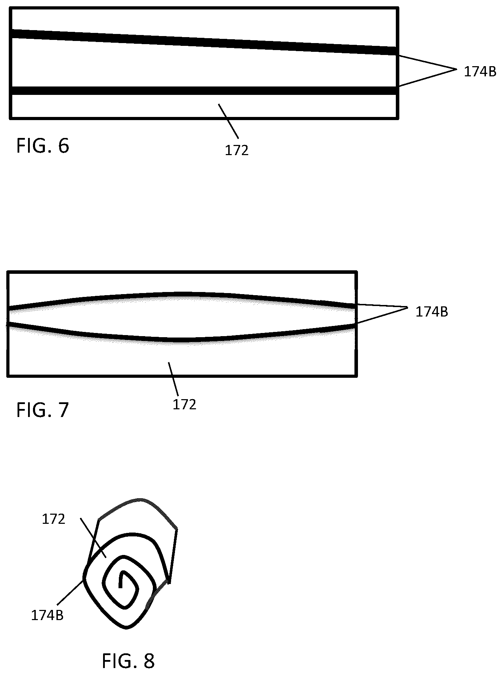

Any number of sheets 174B of graphene 174 may be used. For example, Figure 3 shows three; Figure 5 shows nine; and Figure 6 shows two. Other numbers may be used. Where multiple sheets 174B are used, the sheets 174B are separated from each other by at least part of the polymer framework. The sheets 174B are within the polymer 172, but one or more sheets 174B may be on an edge or surface of the polymer 172 or backing block 170.

The sheets 174B may have any orientation within the backing block 170. The orientations of graphene sheets 174B can be arranged in various ways for optimal thermal and acoustic performance.

Figure 3 shows one implementation. Multiple separate sheets 174B are oriented from a front to a back in the polymer framework. The front is the surface placed adjacent to the array 100, and the back is opposite to the front. The sheets 174B, in cross-section, extend from the front and/or back surfaces of the backing 170 at any angle. In the implementation of Figure 3, the sheets 174B are at different angles. The sheets 174B of graphene 174 fan out in elevation. At the front, a denser concentration of sheets 174B is provided for conducting heat from the array 100. The sheets 174B fan out to better dissipate the heat. The fan may be reversed, such as the sheets 174B closer together on the back surface. The sheets 174B may be shifted, such as further apart, closer together, and/or at different angles than shown. The distribution of sheets 174B may be shifted in the backer, such as one or more (e.g., all) of the sheets 174B being offset in elevation from the center. The sheets 174B are shown as extending generally in thickness (z dimension) and azimuth (x dimension), but may instead be along any dimension (e.g., elevation-thickness or elevation-azimuth).

In the implementation of Figures 4 and 5, the sheets 174B are at the same angle to the front. The sheets 174B are at a 45-85-degree angle from the front surface. Greater or lesser angles may be used, such as 1-44 or 86-90 degrees. The sheets 174B of graphene 174 are tilted to increase attenuation by causing multiple reflections between the sheets 174B. One or more sheets 174B may be at different angles.

Larger or smaller spacing between sheets 174B may be used. Figure 5 shows variation in spacing with a greater density of sheets 174B near a center in elevation. Multiple sheets 174B are at 45-85 degrees from the front surface and have variable spacing across the polymer framework. The sheets 174B of graphene 174 are slightly tilted to increase attenuation with either uniform pitch or finer at the center for more thermal conductivity and pressure apodization (e.g., low impedance at center the center for more reflection).

Figure 6 shows another implementation. Two or more of the sheets 174B are within 20 degrees of parallel with a front of the polymer framework or backing block 170. The two or more of the sheets 174B are each separated from other sheets 174B by at least part of the polymer framework. One of the sheets 174B is substantially parallel (e.g., +/- 2 degrees) with the front. Another one of the sheets 174B is at a non-zero angle to the other sheet and/or the front, such as being at a 3-20-degree angle. For example, two 65 um thick sheets 174B with the sheet closest to the front in the backing block 170 tilted at 3 degrees from parallel is used. The tilt may avoid coherent interference. The two sheets 174B of graphene 174 face each other with an angle to increase absorption in-between the sheets 174B, maximizing the attenuation. For example, compared to an HI-IB, the arrangement of Figure 6 may have greater than 60 dB more attenuation at 2 MHz. Additional sheets 174B, different orientations (angles), different angle separation, and/or different arrangements of sheets 174B may be used. All the sheets 174B may be tilted out of parallel with the front. Multiple sheets 174B may be substantially parallel with the front.

In the implementation of Figure 7, the difference in angle between the sheets 174B of Figure 6 is replaced with one or more of the sheets 174B being curved or non-flat. In any of the implementations or other arrangements, one or more of the sheets 174B may be flat, and/or one or more of the sheets 174B may be curved. The slight bend may increase absorption in-between sheets 174B to maximize the attenuation.

Other arrangements of sheets 174B may be used. For example, the implementation of Figure 6 may be combined with the implementations of Figures 3, 4, or 5. The sheets 174B may be bands that may be weaved. The sheets 174B may have holes or cut-outs for passing other sheets 174B through. The sheets 174B may have lesser extents, so that the sheets 174B can be assembled in various patterns with different angles or directions.

Figure 8 shows another implementation. A single sheet 174B of graphene 174 is rolled into a spiral pattern with the polymer 172 separating the layers of the sheet. For example, the sheet 174B of graphene 174 is coated in polymer 172, and then the coated sheet 174B is rolled. Polymer 172 may be provided outside to provide a desired shape for the backing block 170. In the example of Figure 8, the roll may be sliced in cross-section to form the backing block 170, such that the sheet 174B extends from the front to the back of the backing block 170. Other orientations may be used.

The roll, before curing, may be squeezed or distorted. The rolled cylinder is deformed in a controlled way to provide angled bias to the inner graphene sheet in the spiral. The distortion may cause the angle along the sheet as rolled to vary such that different distances are provided between layers in the spiral at different depths along the roll or backing block 170 when cured. A larger roll may be used where multiple backing blocks 170 are created by slicing or cutting the roll in cross section at different locations.

For use, the array 100 is electrically connected to the ultrasound system or scanner. A flexible circuit, wire bonds, and/or another electrical connection extends from the ground and signal electrodes. For example, flexible circuit material with pads and metal traces are bonded between the backing block 170 and the array 100 for connection with the signal electrodes. A wire jumper or bond connects the ground electrode positioned by or formed by the matching layer 120 to the flexible circuit material or another wire. A cable connects the ultrasound scanner (e.g., transmit and receive beamformers) to the traces of the flexible circuit.

The array 100 is used to transmit and receive. The backing block 170 reduces the acoustic echo or interference from behind the array 100. By having an acoustic impedance close to the array 100, reflections are reduced. By having greater attenuation, the strength of any reflections is reduced. By having a greater thermal conductivity, the heat of the transducer is reduced, avoiding pauses for cooling and/or patient discomfort.

Figure 9 shows one implementation of a method of forming an ultrasound backing block for an ultrasound transducer. Graphene is used as a filler in a polymer-based backing block. The polymer forms a support structure or matrix supporting graphene.

The method forms the backing block 170 of Figures 1 or 3-8 or another backing block for use with the array 100 of Figure 1 or another array. The method may be used to form other acoustic absorbers for other acoustic transducers.

Additional, different, or fewer acts may be provided. For example, acts for aligning with an array and/or bonding in an array stack are provided. As another example, acts for adding other filler are provided. In yet another example, act 920 is not provided.

The acts are performed in the order shown (e.g., top to bottom or numerical) or another order. For example, act 910 is performed prior to act 900 where the graphene filler is stacked with cured pieces of the polymer.

In act 900, the graphene is combined with polymer. Graphene flakes and/or sheets are combined with the polymer.

In one implementation, a blender, vibrator (e.g., shaker), centrifuge, or other stirring device mixes a composite mixture. For example, the two parts of an epoxy (e.g., a thermoset and thermoplastic of the epoxy) in liquid form and filler are placed into a vat. The filler is graphene flakes with or without other powders or particles. Additional, different, or fewer components may be added. The mixture in the vat is mixed by vibration, rotation, stirring, and/or another application of energy to homogenously distribute the different fillers.

The filler, such the graphene flakes with or without rubber powder (e.g., silicone particles), ceramic powder (e.g., aluminum nitride particles), and/or metal powder (e.g., tungsten or Aluminic particles) are added separately or premixed. The types of filler are added separately in any order or are combined and then added to the epoxy. Any ratios, % volumes, and/or % weight of the different types of filler may be used. The components of the filler are measured and added.

The component ingredients to form the composite backing are mixed. Any mixing process may be used, including the order of adding, period of mixing, speed of mixing, temperature of mixing, pressure of mixing, humidity of mixing, or other controllable mixing characteristic. The mixing results in a slurry of composite or mixture.

A robot, pump, and/or servo-controlled nozzle casts the mixture into a mold for an acoustic backing block. The mixture is poured, injected, extruded, or otherwise placed in the mold. In alternative embodiments, the mixture is manually poured into the mold.

The mold is shaped to form an acoustic absorber. The size and shape are based on the transducer or array for which the backing is being formed. Other parts to be fixed in the backing may also be included in the mold, such as wires and/or brackets. The mold may include indentations, extensions, and/or other shapes for aligning, machining, or using the backer once formed.

In another implementation, sheets of graphene are positioned in a framework by pins, clamps, spacers, and/or fasteners. For example, a clamp holds one end of a sheet, which is then placed around one or more pegs or rollers and held by another clamp to form two or more sheets in a mold for forming the backer. As another example, separate sheets are clamped in the desired orientations. The framework forms or includes the mold. The polymer, in liquid form, is poured or injected into the framework so that the polymer fills gaps between and/or around the sheets of graphene. This process combines the polymer with or without another filler and the sheets of graphene.

In yet another implementation, the sheets of graphene are combined with the polymer by stacking the sheets with cured polymer. Multiple pieces of cured polymer molded or cut form portions of the backing block. The pieces are then stacked with the sheets for form the backing. For example, three pieces of cured polymer are formed, one flat and two with 3-degree angles as wedges, as shown in Figure 3. These pieces are stacked with two sheets of graphene as shown in Figure 3. Additional polymer, clamping, and/or other fastening may be used to connect the stack together and/or hold the sheets in place with the pieces of cured polymer. In one example, the stack is laminated, such as potted together.

In act 910, the polymer is cured. The mixture as cast into the mold is cured. The mold may be enclosed, such as covering an open top with a plate. The mold may be held still or moved (e.g., vibrated) during curing.

The curing is by chemical activation. The epoxy cures, becoming more solid or converting from a liquid form to a gel or solid form. Alternatively, or additionally, heat, pressure, or other energy is added to speed the curing. In one implementation, a clamp applies pressure to a top plate to assure size and flatness over 12 hours of curing at 70° Celsius. Other approaches, temperatures, or periods may be used. Variation in any curing characteristics over time as the slurry cures may be used.

After curing, the backing composite is removed from the mold. The backing may be machined, such as sanded, planed, cut, drilled, stamped, and/or otherwise altered for use. For example, a single plate of cured composite may be cut into multiple backing blocks for multiple transducers. Alternatively, a given cured composite from the mold is used for a single or given transducer.

Where the cured polymer is to be stacked, multiple pieces of cured polymer are shaped through molding and/or machining for stacking. Where the combination with graphene occurs prior to curing (e.g., mixing graphene flakes with the liquid polymer), the cured polymer is a composite that includes the graphene. The machining is used to form the backing block.

The polymer as cured provides a support matrix supporting the graphene flakes or sheets. The graphene, as sheets or flakes, is supported by, filler in, and/or held in place by the cured polymer. The polymer is a matrix for the polymer-based composite or backing block where the graphene is the filler.

In act 920, the backing block with graphene is positioned against or in a transducer stack (e.g., position against flexible circuit material providing the signal electrodes for the elements of the array or against the PZT layer as plated). Using epoxy, clamping, and/or other connection, the backing is held in place against the transducer stack. The elements may be formed after or before stacking and bonding of the array layer with the backing block.

Once the transducer is formed (e.g., after bonding of the acoustic stack including the backing block and any kerfing), the array may be used for ultrasound scanning and imaging. Due to the acoustic impedance, acoustic attenuation, and thermal conductivity, the transducer may be used with additional power (e.g., overheats less rapidly) while providing sufficient noise reduction (e.g., absorbs undesired acoustic energy) and while avoiding reflection (e.g., impedance generally matches the transducer element). The graphene filler provides for the desired or tuned characteristics of the backing.

Below are Illustrative Embodiments. These Illustrative Embodiments summarize various aspects or features. The different Illustrative Embodiments may be combined as provided below or in other combinations. Aspects or features of one type (e.g., system, backing, or method) may be combined with or used with another type.

Illustrative Embodiment 1. A transducer array system comprising: an array of ultrasound transducer elements; and a backing comprising a polymer framework with one or more sheets of graphene supported by the polymer framework.

Illustrative Embodiment 2. The transducer array system of Illustrative Embodiment 1 wherein the polymer framework comprises cured epoxy.

Illustrative Embodiment 3. The transducer array system of any of Illustrative Embodiments 1-2 wherein the polymer framework comprises a polymer-based composite with the graphene potted inside the polymer framework.

Illustrative Embodiment 4. The transducer array system of any of Illustrative Embodiments 1-3 wherein the polymer framework comprises a continuous matrix of polymer with the integrated graphene being within the continuous matrix.

Illustrative Embodiment 5. The transducer array system of any of Illustrative Embodiments 1-4 wherein the backing further comprises a metal powder supported in the polymer framework.

Illustrative Embodiment 6. The transducer array system of any of Illustrative Embodiments 1-5 wherein the one or more sheets comprises at least two of the sheets separated from each other by at least part of the polymer framework, each of the sheets having multiple layers of the graphene.

Illustrative Embodiment 7. The transducer array system of any of Illustrative Embodiments 1-6 wherein the one or more sheets comprises multiple separate sheets oriented from a front to back in the polymer framework, the front being adjacent to the array and the back being opposite.

Illustrative Embodiment 8. The transducer array system of Illustrative Embodiment 7 wherein the multiple separate sheets are at different angles to the front.

Illustrative Embodiment 9. The transducer array system of any of Illustrative Embodiments 7-8 wherein the multiple separate sheets are 45-85 degrees from the front.

Illustrative Embodiment 10. The transducer array system of any of Illustrative Embodiments 7-9 wherein the multiple separate sheets are 45-85 degrees from the front and have variable spacing across the polymer framework.

Illustrative Embodiment 11. The transducer array system of any of Illustrative Embodiments 1-11 wherein the one or more sheets comprises two or more of the sheets being within 20 degrees of parallel with a front of the polymer framework, the front being adjacent to the array, the two or more of the sheets being separated by at least part of the polymer framework.

Illustrative Embodiment 12. The transducer array system of Illustrative Embodiment 11 wherein a first of the two or more of the sheets being at a non-zero angle to a second of the two or more of the sheets and/or being non-flat.

Illustrative Embodiment 13. The transducer array system of any of Illustrative Embodiments 1-12 wherein the one or more sheets comprises a first sheet rolled into a spiral pattern.

Illustrative Embodiment 14. An ultrasound imaging probe comprising: a probe housing; a lens attached to the probe housing; one or more matching layers attached to the lens and within the probe housing; and the transducer array system of any of Illustrative Embodiments 1-13 attached to the matching layer and within the probe housing.

Illustrative Embodiment 15. An ultrasound imaging system comprising: an input device, such as a keyboard or touch screen; the ultrasound imaging probe of Illustrative Embodiment 14; a display device; and one or more processors in communication with the input device, the ultrasound imaging probe, and the display device, where the one or more processors receives input from the input device and signals from the ultrasound imaging probe and causes the display device to provide feedback to a user based on the input and the signals.

Illustrative Embodiment 16. A method for forming an ultrasound backing block, the method comprising: combining graphene sheets and a polymer; and curing the polymer as combined with the graphene flakes or sheets, the polymer as cured comprising a support matrix supporting the graphene flakes or sheets.

Illustrative Embodiment 17. The method of Illustrative Embodiment 16 wherein combining comprises supporting the graphene sheets and pouring the polymer as a fluid around the graphene sheets, and wherein curing comprises curing the polymer as supporting the graphene sheets.

Illustrative Embodiment 18. The method of any of Illustrative Embodiments 16-17 wherein combining comprises stacking the graphene sheets with the cured polymer.

Illustrative Embodiment 19. An ultrasound transducer backing block comprising: a matrix of cured polymer, the cured polymer comprising soft polymer; and graphene in the matrix, the graphene held in place in the matrix by the cured polymer around the graphene, the graphene comprising flakes.

Illustrative Embodiment 20. The ultrasound transducer backing block of Illustrative Embodiment 19 wherein a majority of the flakes are 400-500 um along a maximum dimension.

Independent of the grammatical term usage, individuals with male, female or other gender identities are included within the term.

While the invention has been described above by reference to various embodiments, it should be understood that many changes and modifications can be made without departing from the scope of the invention. It is therefore intended that the foregoing detailed description be regarded as illustrative rather than limiting, and that it be understood that it is the following claims, including all equivalents, that are intended to define the spirit and scope of this invention.

## Claims

1. A transducer array (100) system comprising:
an array (100) of ultrasound transducer elements; and
a backing (170) comprising a polymer framework (172) with one or more sheets (174B) of graphene (174) supported by the polymer framework (172).

2. The transducer array (100) system of claim 1 wherein the polymer framework (172) comprises cured epoxy.

3. The transducer array (100) system of claim 1 or 2 wherein the polymer framework (172) comprises a polymer-based composite with the graphene (174) potted inside the polymer framework (172).

4. The transducer array (100) system of one of claims 1 to 3 wherein the polymer framework (172) comprises a continuous matrix of polymer with the integrated graphene (174) being within the continuous matrix.

5. The transducer array (100) system of one of claims 1 to 4 wherein the backing (170) further comprises a metal powder supported in the polymer framework (172).

6. The transducer array (100) system of one of claims 1 to 5 wherein the one or more sheets (174B) comprises at least two of the sheets (174B) separated from each other by at least part of the polymer framework (172), each of the sheets (174B) having multiple layers of the graphene (174).

7. The transducer array (100) system of one of claims 1 to 6 wherein the one or more sheets (174B) comprises multiple separate sheets (174B) oriented from a front to back in the polymer framework (172), the front being adjacent to the array (100) and the back being opposite.

8. The transducer array (100) system of claim 7 wherein the multiple separate sheets (174B) are at different angles to the front, wherein the multiple separate sheets (174B) particularly are 5-45 degrees from the front, wherein the multiple separate sheets (174B) particularly are 5-20 degrees from the front and have variable spacing across the polymer framework (172).

9. The transducer array (100) system of one of claims 1 to 8 wherein the one or more sheets (174B) comprises two or more of the sheets (174B) being within 20 degrees of parallel with a front of the polymer framework (172), the front being adjacent to the array (100), the two or more of the sheets (174B) being separated by at least part of the polymer framework (172), wherein a first of the two or more of the sheets (174B) particularly being at a non-zero angle to a second of the two or more of the sheets (174B) and/or being non-flat.

10. The transducer array (100) system of one of claims 1 to 9 wherein the one or more sheets (174B) comprises a first sheet (174B) rolled into a spiral pattern.

11. A method for forming an ultrasound backing (170) block, the method comprising:
combining (900) graphene (174) sheets (174B) and a polymer; and
curing (910) the polymer, the polymer as cured comprising a support matrix supporting the graphene (174) flakes or sheets (174B).

12. The method of claim 11 wherein combining (900) comprises supporting the graphene (174) sheets (174B) and pouring the polymer as a fluid around the graphene (174) sheets (174B), and wherein curing (910) comprises curing (910) the polymer as supporting the graphene (174) sheets (174B).

13. The method of claim 12 wherein combining (900) comprises stacking the graphene (174) sheets (174B) with the cured polymer.

14. An ultrasound transducer backing (170) block comprising:
a matrix of cured polymer (172), the cured polymer (172) comprising soft polymer; and
graphene (174) in the matrix, the graphene (174) held in place in the matrix by the cured polymer (172) around the graphene (174), the graphene (174) comprising flakes.

15. The ultrasound transducer backing (170) block of claim 14 wherein a majority of the flakes are 400-500 um along a maximum dimension.
